# EUROPÄISCHE PATENTANMELDUNG

(11) **EP 0 941 987 A1**
(43) Veröffentlichungstag der Anmeldung: **15.09.1999**
(21) Anmeldenummer: 99103877.9
(22) Anmeldetag: 01.03.1999
(51) Int. Cl.: C07C 221/00, C07C 225/22, C07C 205/45, C07C 45/46, C07C 49/84

(54) **Verfahren zur Herstellung von 2-Amino-4,5,3',4'-tetramethoxybenzophenon**

(30) Priorität: 13.03.1998 CH 61098
(71) Anmelder: LONZA A.G., CH-4002 Basel (CH)
(72) Erfinder: Werbitzky, Oleg, Dr., 3930 Visp (CH); Heinzmann, Etienne, 3932 Visperterminen (CH); Brieden, Walter, Dr., 3902 Brig-Glis (CH)

(57) **Zusammenfassung**

2-Amino-4,5,3',4'-tetramethoxybenzophenon der Formel 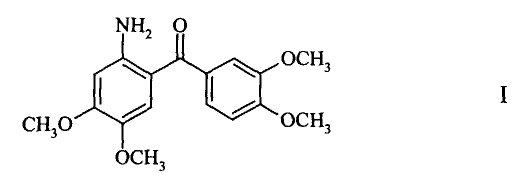 wird durch Mononitrierung von 3,3',4,4'-Tetramethoxybenzophenon mit Salpetersäure in Gegenwart von Essigsäure und anschliessende Reduktion der Nitrogruppe hergestellt. Die Verbindung ist ein Zwischenprodukt in der Synthese von Wirkstoffen gegen rheumatoide Arthritis.

## Beschreibung

Die vorliegende Erfindung betrifft ein Verfahren zur Herstellung von 2-Amino-4,5,3',4'-tetramethoxybenzophenon der Formel

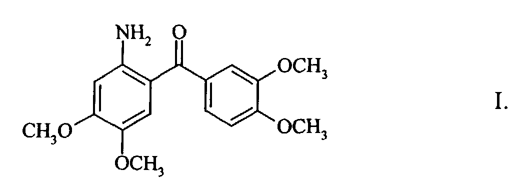

Diese Verbindung ist ein wichtiges Zwischenprodukt in der Synthese von Chinolin-Derivaten, welche als pharmazeutische Wirkstoffe gegen rheumatoide Arthrithis von Bedeutung sind (EP-A 0 567 107, EP-A 0 608 870, EP-A 0 634 169, EP-A 0 686 630, WO-A 95/24394, WO-A 97/09984). Ein Herstellungsverfahren für diese Verbindung wurde bisher jedoch nicht publiziert.

Aufgabe der vorliegenden Erfindung war daher, ein im technischen Massstab durchführbares Verfahren zur Herstellung von 2-Amino-4,5,3',4'-tetramethoxybenzophenon bereitzustellen.

Erfindungsgemäss wird diese Aufgabe durch das Verfahren gemäss Patentanspruch 1 gelöst.

Es wurde überraschend gefunden, dass das gewünschte Produkt durch selektive (Mono-) Nitrierung von 3,3',4,4'-Tetramethoxybenzophenon der Formel 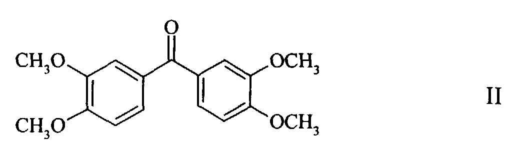 mit Salpetersäure in Gegenwart von Essigsäure zu 2-Nitro-4,5,3',4'-tetramethoxybenzophenon der Formel 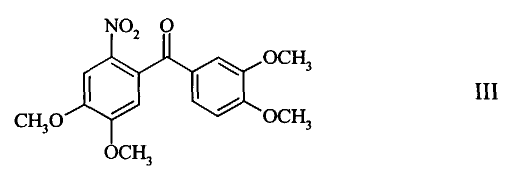 und anschliessende selektive Reduktion der Nitrogruppe in guter Ausbeute erhältlich ist. Diese Synthese ist deswegen überraschend, weil bekannt ist, dass bei der Nitrierung von 3,3',4,4'-Tetramethoxybenzophenon mit Salpetersäure unter üblichen Bedingungen in praktisch quantitativer Ausbeute die zweifache Nitrierung zu 2,2'-Dinitro-4,5,4',5'-tetramethoxybenzophenon erfolgt (W. Lawson et al., *J. Chem. Soc*. **1924**,*125*, 626). In Gegenwart von Essigsäure wird dagegen mit hoher Selektivität das mononitrierte Produkt 2-Nitro-4,5,3',4'-tetramethoxybenzophenon (III) erhalten. Diese neue Verbindung ist ebenfalls Gegenstand der vorliegenden Erfindung.

Die Nitrierung wird vorzugsweise mit 65%iger ("konzentrierter") Salpetersäure in Essigsäure ("Eisessig") als Lösungsmittel bei 0 bis 40 °C durchgeführt. Besonders bevorzugt ist eine Reaktionstemperatur von 10 bis 30 °C.

Die Reduktion der Nitrogruppe in III zur Aminogruppe kann unter den üblichen Bedingungen erfolgen, beispielsweise mit Zinn und Salzsäure. Es sind lediglich solche Bedingungen zu vermeiden, unter denen mit der Reduktion der Ketogruppe zu rechnen ist.

Vorzugsweise wird die Reduktion durch katalytische Hydrierung durchgeführt.

Als Katalysator für die katalytische Hydrierung ist ein Platin-Trägerkatalysator besonders bevorzugt, insbesondere Platin auf Aktivkohle.

Die Herstellung von 3,3',4,4'-Tetramethoxybenzophenon ist aus der Literatur bekannt und wird üblicherweise durch Friedel-Crafts-Acylierung von 1,2-Dimethoxybenzol mit 3,4-Dimethoxybenzoylchlorid durchgeführt (W. Lawson et al., loc. cit.). Eine andere bekannte Synthese dieser Verbindung verläuft über die Kondensation von Oxalylchlorid mit 1,2-Dimethoxybenzol (H. Staudinger et al., *Helv. Chim*. *Acta* **1921**, *4*, 334). Beide bekannten Synthesen verwenden Schwefelkohlenstoff als Lösungsmittel und stöchiometrische Mengen von Aluminiumchlorid als Katalysator und sind somit für eine grosstechnische Produktion aus sicherheitstechnischen Gründen und wegen der grossen Abfallmengen nicht geeignet.

Überraschend wurde gefunden, dass man bei Verwendung von Polyphosphorsäure als Katalysator 1,2-Dimethoxybenzol (Veratrol) direkt mit 3,4-Dimethoxybenzoesäure (Veratrumsäure) acylieren kann. Die Vorteile dieser Reaktion liegen neben der guten Zugänglichkeit der Ausgangsmaterialien in einer hohen Ausbeute und einer sehr einfachen Produktisolierung. Nach Zugabe von Wasser kann das 3,3',4,4'-Tetramethoxybenzophenon nämlich durch eine einfache Filtration isoliert werden.

Die folgenden Beispiele verdeutlichen die Durchführung des erfindungsgemässen Verfahrens, ohne dass darin eine Einschränkung zu sehen ist.

### Beispiel 1

### 3,3',4,4'-Tetramethoxybenzophenon

In 100 g Polyphosphorsäure (*d* = 2,1 g/ml) wurden 16,92 g (120 mmol) 1,2-Dimethoxybenzol und 22,08 g (120 mmol) 3,4-Dimethoxybenzoesäure 30 min bei 80 °C gerührt. Dann wurde das Reaktionsgemisch auf 60 °C abgekühlt und innerhalb von 30 min tropfenweise mit 250 ml Wasser versetzt. Das so erhaltene Gemisch wurde mit Eis weiter abgekühlt, der entstandene Niederschlag abfiltriert, zweimal mit je 60 ml Wasser gewaschen und schliesslich im Vakuum getrocknet. Man erhielt 35,7 g eines rötlichen Produktes.
Ausbeute: 98% der Theorie.

### Beispiel 2

### 2-Nitro-4,5,3',4'-tetramethoxybenzophenon

Ein Gemisch aus 2,93 g (9,7 mmol) 3,3',4,4'-Tetramethoxybenzophenon (hergestellt nach Beispiel 1), 9 g Eisessig und 1,88 g (19,4 mmol) 65%iger Salpetersäure wurde 7,5 h bei Raumtemperatur gerührt. Danach wurden 22 ml Wasser zugetropft, der entstandene Niederschlag abfiltriert, zweimal mit je 6 ml Wasser gewaschen und getrocknet. Man erhielt 2,41 g eines beigen Feststoffes. Ausbeute: 71,5% der Theorie.
¹H NMR (CDCl₃, 400 MHz): δ = 7,72 (s, 1H); 7,59 (d, *J* = 2,1 Hz, 1H); 7,05 (dd, *J* = 8,4/2,1 Hz, 1H); 6,85 (s, 1H); 6,78 (d, *J* = 8,4 Hz, 1H); 4,03 (s, 3H); 3,97 (s, 3H); 3,95 (s, 3H); 3,92 (s, 3H).
¹³C NMR ("off resonance", CDCl₃, 100 MHz): δ = 192 (s); 153,9 (s); 153,8 (s); 149,6 (s); 149,5 (s); 139,6 (s); 130,6 (s); 129,5 (s); 124,5 (d); 110,4 (d); 110,1 (d); 110,1 (d); 106,9 (d); 56,7 (q); 56,6 (q); 56,1 (q); 56,1 (q).

### Beispiel 3

### 2-Amino-4,5,3',4'-tetramethoxybenzophenon

In 10 ml Ethanol wurden 1 g (2,88 mmol) 2-Nitro-4,5,3',4'-tetramethoxybenzophenon (hergestellt nach Beispiel 2) an 100 mg Platin/Aktivkohle (5% Pt, feucht, H₂O-Gehalt 50%) bei 40 °C und 2 bar Wasserstoffdruck hydriert. Nach 5 h wurde das Reaktionsgemisch auf Raumtemperatur abgekühlt und mit 10 ml Acetonitril versetzt. Der Katalysator wurde abfiltriert und das Filtrat im Vakuum eingeengt. Man erhielt 0,88 g 2-Amino-4,5,3',4'-tetramethoxybenzophenon als beigen Feststoff.
Ausbeute: 96% der Theorie.

## Patentansprüche

1. Verfahren zur Herstellung von 2-Amino-4,5,3',4'-tetramethoxybenzophenon der Formel 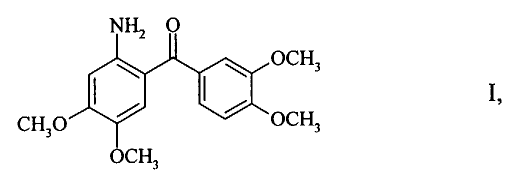 dadurch gekennzeichnet, dass in einer ersten Stufe 3,3',4,4'-Tetramethoxybenzophenon der Formel 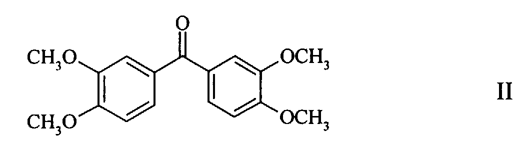 mit Salpetersäure in Gegenwart von Essigsäure zu 2-Nitro-4,5,3',4'-tetramethoxybenzophenon der Formel 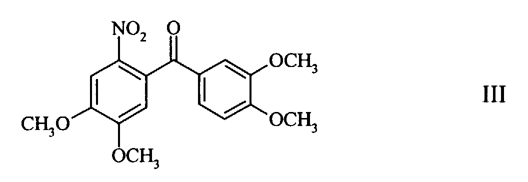 nitriert und in einer zweiten Stufe zur Zielverbindung reduziert wird.

2. Verfahren nach Anspruch 1, dadurch gekennzeichnet, dass die Nitrierung mit 65%iger Salpetersäure in Essigsäure als Lösungsmittel bei 0 bis 40 °C durchgeführt wird.

3. Verfahren nach Anspruch 1 oder 2, dadurch gekennzeichnet, dass die Reduktion durch katalytische Hydrierung erfolgt.

4. Verfahren nach Anspruch 3, dadurch gekennzeichnet, dass die katalytische Hydrierung an einem Platin-Trägerkatalysator durchgeführt wird.

5. Verfahren nach Anspruch 4, dadurch gekennzeichnet, dass als Platin-Trägerkatalysator Platin auf Aktivkohle eingesetzt wird.

6. Verfahren zur Herstellung von 3,3',4,4'-Tetramethoxybenzophenon der Formel 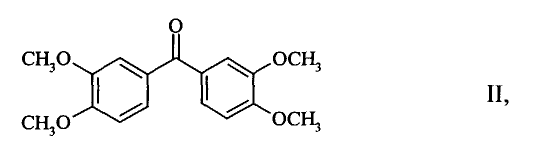 dadurch gekennzeichnet, dass 1,2-Dimethoxybenzol mit 3,4-Dimethoxybenzoesäure in Gegenwart von Polyphosphorsäure acyliert wird.

7. 2-Nitro-4,5,3',4'-tetramethoxybenzophenon der Formel
